(19) **Europäisches Patentamt / European Patent Office / Office européen des brevets**

(11) **EP 1 651 183 B1**

(12) **EUROPÄISCHE PATENTSCHRIFT**

(45) Veröffentlichungstag und Bekanntmachung des Hinweises auf die Patenterteilung:
**02.04.2008 Patentblatt 2008/14**

(21) Anmeldenummer: **04734243.1**

(22) Anmeldetag: **21.05.2004**

(51) Int Cl.:
***A61K 8/97*** *(2006.01)*     ***A61Q 19/00*** *(2006.01)*

(86) Internationale Anmeldenummer:
**PCT/EP2004/005542**

(87) Internationale Veröffentlichungsnummer:
**WO 2004/105706 (09.12.2004 Gazette 2004/50)**

(54) **KOSMETIKA ENTHALTEND WIRKSTOFFZUBEREITUNG MIT PFLANZENEXTRAKTEN**

COSMETIC PRODUCTS COMPRISING ACTIVE INGREDIENT COMPOSITION COMPRISING VEGETABLE EXTRACTS

PRODUITS COSMETIQUES CONTENANT UNE PREPARATION DE PRINCIPE ACTIF CONTENANT DES EXTRAITS VEGETAUX

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.05.2003 DE 10325156**

(43) Veröffentlichungstag der Anmeldung:
**03.05.2006 Patentblatt 2006/18**

(73) Patentinhaber: **Coty B.V.**
**2031 CC Haarlem (NL)**

(72) Erfinder:
• **GOLZ-BERNER, Karin**
**MC-98000 Monaco (MC)**

• **ZASTROW, Leonhard**
**MC-98000 Monaco (MC)**

(74) Vertreter: **Walter, Wolf-Jürgen**
**Anwaltskanzlei**
**Gulde Hengelhaupt Ziebig & Schneider**
**Wallstrasse 58/59**
**10179 Berlin (DE)**

(56) Entgegenhaltungen:
**WO-A-01/26617**      **WO-A-99/66881**

Bemerkungen:
Die Akte enthält technische Angaben, die nach dem Eingang der Anmeldung eingereicht wurden und die nicht in dieser Patentschrift enthalten sind.

EP 1 651 183 B1

**Beschreibung**

Die Erfindung betrifft eine Wirkstoffzubereitung für Kosmetika, die Pflanzenextrakte enthält und besondere antiradikalische Eigenschaften hat.

Aus der WO 99/66881 ist eine kosmetische Wirkstoffzubereitung mit hohem Radikalschutzfaktor bekannt, die als Hauptbestandteile einen in Mikrokapseln eingeschlossenen Rindenextrakt von Quebracho blanco und einen Seidenraupenextrakt in einem Gel zusammen mit Phospholipiden enthält und einen Assoziationskomplex in dem Gel bildet. Dieser Wirkstoffkomplex kann zusätzlich weitere Bestandteile enthalten, beispielsweise Pflanzenextrakte. Bei Pflanzenextrakten wurden unter vielen anderen auch die von Kaffeebohnen und Angelikawurzel genannt. Es werden mit diesen Kombinationen Radikalschutzfaktoren von 100 bis 10000 erreicht und je nach Anteil der Wirkstoffzubereitung werden in der kosmetischen Zubereitung Radikalschutzfaktoren von 40-200 erreicht. Die WO 01/26617 beschreibt ein ähnliches Gemisch, das noch zusätzlich ein Hefeaufschlussprodukt und Cyclodextrine enthält. Beiden gemeinsam ist die Verkapselung des Basiswirkstoffe in Liposomen.

Der Erfindung liegt die Aufgabe zugrunde, ohne Einsatz von verkapselnden Liposomen eine einfach herstellbare Zusammensetzung zur kosmetischen Anwendung bereitzustellen, die ebenfalls einen hohen Radikalschutzfaktor zeigt, sich aber deutlich einfacher mit anderen kosmetischen Bestandteilen verarbeiten läßt und auch die Herstellung von Parfüms und Sprays zuläßt.

Erfindungsgemäß ist die kosmetische Zubereitung mit Pflanzenextrakten dadurch gekennzeichnet, daß sie 0,1 bis 10 Gew-% einer Wirkstoffzubereitung umfasst, enthaltend ein liposomenfreies Gemisch von Pflanzenextrakten auf alkoholischer Basis, bestehend aus 0,1 bis 2 Gew-% Extrakt grüner Kaffeebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen $C_2$-$C_5$-Alkohol, wobei die Konzentration der Wirkstoffzubereitung auf das Gesamtgewicht der kosmetischen Zubereitung bezogen ist, und wobei die Wirkstoffzubereitung einen Radikalschutzfaktor im Bereich von 1400-2900 x $10^{14}$ Radikale pro mg hat.

Die Extrakte sind alkoholische oder wäßrig-alkoholische Extrakte; vorzugsweise sind es alkoholische Extrakte. Die Extraktionstemperaturen liegen zwischen 18 und 28°C. Bei Pongamia pinnata wurde die Gesamtpflanze extrahiert.

Dieses Extraktgemisch kann in einem Anteil von 0,1 bis 10 Gew-%, vorzugsweise 0,1 bis 5 Gew-%, in einem Kosmetikum enthalten sein, bezogen auf das Gesamtgewicht des Kosmetikums. Es wurde gefunden, daß ein solches Wirkstoffgemisch einen unerwartet hohen Radikalschutzfaktor (RPF) von etwa 1400-2900 x $10^{14}$ Radikale pro mg zeigt, gemessen durch Bestimmung der Anzahl freier Radikale einer Lösung einer Testsubstanz ($S_1$) mittels Elektronenspinresonanz (ESR) im Vergleich mit dem ESR-Meßergebnis der kosmetischen Wirkstoffzubereitung nach der Beziehung

$$\text{RPF} = (\text{RC} \times \text{RF}) / \text{PI}$$

worin RF = ($S_1$-$S_2$) / $S_1$ ; RC = Konzentration der Testsubstanz (Radikale/ml); PI = Konzentration der Wirkstoffzubereitung (mg/ml) (Messung gemäß WO 99/66881).

Dieser gefundene RPF ist deutlich höher als der einer Wirkstoffzubereitung in WO 99/66881, der mit 1255 angegeben wurde.

Es wurde weiterhin gefunden, daß die erfindungsgemäße Wirkstoffzubereitung in einem bevorzugten Konzentrationsbereich von 0,5 bis 2 Gew-% Anteil in einer kosmetischen Zusammensetzung zu Radikalschutzfaktoren der kosmetischen Zusammensetzung von 60 bis 140 x $10^{14}$ Radikale pro mg führt, was deutlich über den in der WO 99/66881 in den Beispielen genannten Werten von 35 bis 49 x $10^{14}$ liegt.

Die Verwendung der erfindungsgemäßen Wirkstoffzubereitung kann in W/O- oder O/W-Emulsionen, Gelen oder Gel-Emulsionen erfolgen. Besonders vorteilhaft ist die Verwendung in Parfüms oder Sprays. Da die aus WO 99/66881 bekannten Wirkstoffzubereitungen stets mit einem Gel verbunden sind und darüber hinaus die Wirkstoffe in Liposomen verkapselt sind, ist es häufig sehr schwierig, derartige Formulierungen zu zerstäuben, so daß ein solcher Anwendungsbereich für diese Formulierungen mit hohen Radikalschutzfaktoren regelmäßig verschlossen bleibt. Demgegenüber ist die alkoholische Lösung der erfindungsgemäßen Wirkstoffzubereitung weniger aufwendig in der Herstellung, da die Liposomenherstellung entfällt, sie zeigt hohe Radikalschutzfaktoren und sie ist problemlos in Spray- oder Parfümanwendungen einzubringen und vom Anwender zu entnehmen.

Die erfindungsgemäße Wirkstoffzubereitung kann auch mit anderen kosmetischen Hilfs- und Wirkstoffen kombiniert und zu entsprechenden Anwendungsformen verarbeitet werden.

Zu derartigen Hilfsstoffen gehören Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Stabilisatoren.

Zu den kosmetischen Wirkstoffen gehören z. B. anorganische und organische Lichtschutzmittel, weitere Radikalfänger, Feuchthaltemittel, Vitamine, Enzyme, weitere pflanzliche Wirkstoffe, Polymere, Melanin, Antioxidationsmittel, entzündungswidrige natürliche Wirkstoffe.

Die Erfindung soll nachstehend durch Beispiele näher erläutert werden. Alle Angaben erfolgen in Gewichtsprozent, sofern nichts anderes angegeben ist.

Beispiel 1 **Feuchtigkeits-Hautbalsam**

| Phase A | |
|---|---|
| Wasser | q.s. ad 100 |
| Glycerin | 2,0 |
| Butylenglycol | 2,0 |
| Tetranatrium-ethylendiaminessigsäure | 0,1 |
| Konservierungsmittel | 0,4 |
| pH-Regulator | 0,3 |
| **Phase B** | |
| Beheneth-25 | 3,3 |
| Cetearylalkohol | 2,7 |
| Dicaprylcarbonat | 8,5 |
| Shea Butter | 7,2 |
| Phenoxyethanol | 0,9 |
| modifizierter Maisstärkepuder | 3,0 |
| Dimethicone | 1,4 |
| Simulgel® NS . | 3,5 |
| **Phase C** | |
| Farben | 0,1 |
| Wasser vulkanischen Ursprungs** | 1,0 |
| Peptid Palmitoyl-Gly-His-Lys | 0,5 |
| alkoholisches Pflanzenextraktgemisch* | 0,2 |
| Crithmum maritinum-Extrakt | 0,5 |
| hydrolysiertes Sojaprotein | 1,0 |
| Benzophenone-4 (für Farben) | 0,4 |
| * aus 0,2 Gew-% Kaffeebohnensamen, 0,2 Gew-% Camellia sinensis Blättern, 0,2 Gew-% Ponagamia pinnata, 0,2 Gew-% Angelikawurzel und 99,8 Gew-% Ethanol. | |
| ** mit folgenden Salzkonzentrationen: 0,01 - 0,05 mg/l Fe, 100 - 300 mg/l K, 1000-2000 mg/l Na, 80 - 200 mg/l Mg, 50 - 150 mg/l Ca, 50 bis 150 mg/l Si (als $SiO_2$), 0,01 bis 0,1 mg/l P, 0,001 - 0,005 mg/l Se, 0,01 - 0,03 mg/l Zn. | |

Die Phasen A und B werden separat bei etwa 60 °C gemischt und mit der bei ca. 35 °C gemischten Phase C unter Rühren bei etwa 35 °C zusammengeführt.
Der RPF des Hautbalsams beträgt 68 (x $10^{14}$ Radikale pro mg).

Beispiel 2 **Parfüm**

| Ethanol | q.s. ad 100 |
|---|---|
| alkoholisches Pflanzenextraktgemisch* | 9,5 |
| Parfüm | 8 |
| RFP = 137. | |

<u>Beispiel 3</u> **Spray**

| Ethanol | q.s. ad 100 |
| alkoholisches Pflanzenextraktgemisch* | 5 |
| Treibgas | 38 |
| RPF = 93. | |

Der Spray ließ sich ausgezeichnet handhaben, zeigte eine sehr feine Tröpfchenverteilung und ergab keinerlei Probleme wie Vergleichssprays, in denen Pflanzenextrakte in Liposomen verkapselt vorlagen.

**Patentansprüche**

1. Kosmetische Zubereitung mit Pflanzenextrakten, **dadurch gekennzeichnet, daß** sie 0,1 bis 10 Gew-% einer Wirkstoffzubereitung umfasst, enthaltend ein liposomenfreies Gemisch von Pflanzenextrakten auf alkoholischer Basis, bestehend aus 0,1 bis 2 Gew-% Extrakt grüner Kaffeebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen $C_2$-$C_5$-Alkohol, wobei die Konzentration der Wirkstoffzubereitung auf das Gesamtgewicht der kosmetischen Zubereitung bezogen ist, und wobei die Wirkstoffzubereitung einen Radikalschutzfaktor im Bereich von 1400-2900 x $10^{14}$ Radikale pro mg hat.

2. Kosmetische Zubereitung nach Anspruch 1, **dadurch gekennzeichnet, daß** sie eine Wirkstoffzubereitung aus einem Gemisch von Pflanzenextrakten auf alkoholischer Basis umfasst, bestehend aus 0,2 Gew-% Extrakt grüner Kaffeebohnen, 0,2 Gew-% Extrakt von Blättern von Camellia sinensis, 0,2 Gew-% Extrakt von Pongamia pinnata und 0,2 Gew-% Extrakt der Wurzeln von Angelica archangelica und 99,2 Gew-% Ethanol.

3. Kosmetische Zubereitung nach Anspruch 1, **dadurch gekennzeichnet, daß** die Wirkstoffzubereitung in einer Konzentration von 0,1 bis 5 Gew-% vorliegt und der Radikalschutzfaktor der kosmetischen Zubereitung im Bereich von 60-140 x $10^{14}$ Radikale pro mg liegt.

4. Kosmetische Zubereitung nach Anspruch 1, **dadurch gekennzeichnet, daß** sie als Spray oder Parfüm vorliegt.

**Claims**

1. Cosmetic preparation with plant extracts comprising 0.1 and 10% by weight of an active preparation comprising a liposome-free mixture of plant extracts with an alcoholic base, which consists of 0.1 to 2% by weight extract from green coffee beans, 0.1 to 2% by weight extract from the leaves of Camellia sinensis, 0.1 to 2% by weight extract from Pongamia pinnata and 0.1 to 2% by weight extract from the roots of Angelica archangelica, a monovalent $C_2$-$C_5$ alcohol making up the remainder up to 100% by weight, wherein the radical protection factor ranges between 1,400 and 2,900 x $10^{14}$ radicals per mg and wherein the aforesaid concentrations are relative to the total weight of the cosmetic preparation.

2. Cosmetic preparation according to Claim 1, wherein said active preparation is a mixture of plant extracts with an alcoholic base, which consists of 0.2% by weight extract from green coffee beans, 0.2% by weight extract from the leaves of Camellia sinensis, 0.2% by weight extract from Pongamia pinnata and 0.2% by weight extract from the roots of Angelica archangelica and 99.2% by weight ethanol.

3. Cosmetic preparation according to Claim 1, wherein said active preparation is provided in a cosmetic preparation in a concentration ranging between 0.1 and 5% by weight, and the radical protection factor of said cosmetic preparation ranges between 60 and 140 x $10^{14}$ radicals per mg.

4. Cosmetic preparation according to Claim 1, wherein said active preparation is provided in a spray or a perfume.

**Revendications**

1. Préparation cosmétique à extraits de plantes, **caractérisée en ce qu'**elle comprend de 0,1 à 10 % en poids d'une préparation d'agents actifs, contenant un mélange d'extraits de plantes exempt de liposomes à base alcoolique, se composant 0,1 à 2 % en poids d'extrait de grains de café verts, de 0,1 à 2 % en poids d'extraits de feuilles de Camellia sinensis, de 0,1 à 2 % en poids d'extrait de Pongamia pinnata et de 0,1 à 2 % en poids d'extrait de racines d'Angelica archangelica et le résidu représentant jusqu'à 100 % en poids d'un alcool monohydrique en $C_2$-$C_5$, la concentration de la préparation d'agents actifs étant rapportée au poids total de la préparation cosmétique, et la préparation d'agents actifs ayant un facteur de protection contre les radicaux dans le domaine de 1400-2900 x $10^{14}$ radicaux par mg.

2. Préparation cosmétique selon la revendication 1, **caractérisée en ce qu'**elle comprend une préparation d'agents actifs faite d'un mélange d'extraits de plantes à base alcoolique se composant de 0,2 % en poids d'extrait de grains de café verts, de 0,2 % en poids d'extraits de feuilles de Camellia sinensis, de 0,2 % en poids d'extrait de Pongamia pinnata et de 0,2 % en poids d'extrait de racines d'Angelica archangelica et de 99,2 % en poids d'éthanol.

3. Préparation cosmétique selon la revendication 1, **caractérisée en ce que** la préparation d'agents actifs est présente dans une concentration de 0,1 à 5 % en poids et **en ce que** le facteur de protection contre les radicaux de la préparation cosmétique se situe dans le domaine de 60-140 x $10^{14}$ radicaux par mg.

4. Préparation cosmétique selon la revendication 1, **caractérisée en ce qu'**elle est présente sous la forme d'un aérosol ou d'un parfum.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9966881 A
- WO 0126617 A